# EUROPEAN PATENT APPLICATION

(11) **EP 2 606 889 A1**
(43) Date of publication of application: **26.06.2013**
(21) Application number: 12197999.1
(22) Date of filing: 19.12.2012
(51) Int. Cl.: A61K 31/427, A61K 31/506, C07D 417/04, A61P 35/00

(54) **Substituted Thiazoles as VEGFR2 kinase Inhibitors**

(30) Priority: 21.12.2011 US 201161578633 P
(71) Applicant: Telik, Inc., Palo Alto, CA 94304 (US)
(72) Inventor: Beroza, Paul P., Palo Alto, CA California 94304 (US); Damodaran, Komath V., Palo Alto, CA California 94304 (US); Laborde, Edgardo, Palo Alto, CA California 94304 (US); Thomas, William, Palo Alto, CA California 94304 (US)
(74) Representative: Gibson, Mark

(57) **Abstract**

This invention relates to substituted thiazoles of formula I: 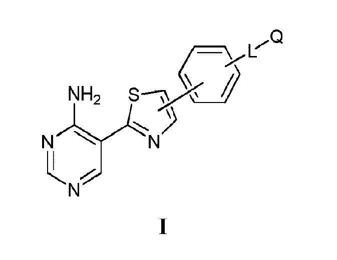 wherein: L is selected from the group consisting of -NHC(X)-, -C(X)NH-, -NHC(X)NH-, -C(X)-, -C(X)NH-alkylene- and -NHC(X)-alkylene-, where X is oxygen or sulfur; and Q is selected from the group consisting of alkyl, substituted alkyl, aryl, substituted aryl, heteroaryl, substituted heteroaryl, cycloalkyl, substituted cycloalkyl, heterocyclic and substituted heterocyclic, pharmaceutically acceptable salt thereof, pharmaceutical compositions containing them, and their use as VEGFR2 (vascular endothelial growth factor receptor 2) kinase inhibitors and in the treatment of diseases capable of treatment by a VEGFR2 kinase inhibitor, particularly cancer.

## Description

### Cross-Reference to Related Application

This application claims the benefit under 35 U.S.C. 119(e) of U.S. Provisional Application No. 61/578,633, filed December 21, 2011 which is incorporated by reference in its entirety into this application.

### Field

This invention relates to substituted thiazoles, their salts, pharmaceutical compositions containing them, and their use as VEGFR2 (vascular endothelial growth factor receptor 2) kinase inhibitors and in the treatment of diseases capable of treatment by a VEGFR2 kinase inhibitor, particularly cancer.

### Background

Vascular endothelial growth factor (VEGF) stimulation of VEGF receptors (VEGFRs) promotes endothelial cell growth, migration, and survival. Inhibition of this pathway with antibodies or small molecules has been proven effective as anticancer therapy. It is desirable to develop novel compounds that are potent inhibitors of VEGFR2 kinase as anticancer agents.

### Summary of the Invention

In one embodiment, this invention provides a compound of formula I: 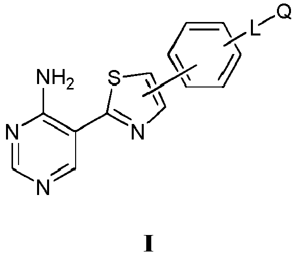 wherein:
L is selected from the group consisting of -NHC(X)-, -C(X)NH-, -NHC(X)NH-, -C(X)-, -C(X)NH-alkylene-, and -NHC(X)-alkylene-, where X is oxygen or sulfur; and
Q is selected from the group consisting of alkyl, substituted alkyl, aryl, substituted aryl, heteroaryl, substituted heteroaryl, cycloalkyl, substituted cycloalkyl, heterocyclic, and substituted heterocyclic,
or a pharmaceutically acceptable salt thereof.

In another embodiment, this invention provides a compound of formula II: 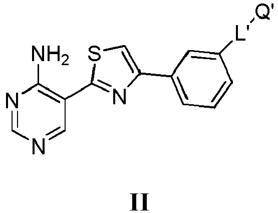 wherein:
L' is selected from the group consisting of -NHC(X)- and -NHC(X)NH-; and
Q' is selected from the group consisting of aryl, substituted aryl, cycloalkyl, and substituted cycloalkyl,
or a pharmaceutically acceptable salt thereof.
In another embodiment, this invention provides a compound selected from the compounds listed in Tables 1-4.

### Detailed Description

### Definitions and General Parameters

Unless defined otherwise, all technical and scientific terms used herein have the same meaning as commonly understood by one of ordinary skill in the art to which this invention belongs. As used herein the following terms have the following meanings.

As used herein, the term "comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition consisting essentially of the elements as defined herein would not exclude other materials or steps that do not materially affect the basic and novel characteristic(s) of the claimed invention. "Consisting of" shall mean excluding more than trace elements of other ingredients and substantial method steps. Embodiments defined by each of these transition terms are within the scope of this invention.

The term "about" when used before a numerical designation, e.g., temperature, time, amount, and concentration, including range, indicates approximations which may vary by (+) or (-)10%, 5% or 1%.

"Alkyl" refers to linear or branched saturated aliphatic hydrocarbon groups with one point of attachment, containing 1 to 10 carbon atoms, preferably 1 to 6 carbon atoms. Alkyl groups include, but are not limited to, methyl; ethyl; propyls such as propan-1-yl, propan-2-yl (iso-propyl); butyls such as butan-1-yl, butan-2-yl (*sec*-butyl), 2-methyl-propan-1-yl (*iso*-butyl), 2-methyl-propan-2-yl (t-butyl), and the like. An alkyl group comprises from 1 to 10 carbon atoms, e.g., from 1 to 6 carbon atoms.

"Alkenyl" refers to an aliphatic group containing at least one carbon-carbon double bond and having from 2 to about 10 carbon atoms, e.g., from 2 to 6 carbon atoms or 2 to 4 carbon atoms and having at least one site of vinyl unsaturation (>C = C<). Alkenyl groups include ethenyl, propenyl, 1,3-butadienyl, and the like.

"Alkynyl" refers to an aliphatic group containing at least one carbon-carbon triple bond. The term "alkynyl" is also meant to include those groups having one triple bond and one double bond.

"Alkoxy" refers to the group -O-alkyl, wherein the alkyl group may be optionally substituted. Alkoxy includes, by way of example, methoxy, ethoxy, n-propoxy, isopropoxy, *n*-butoxy, t-butoxy, *sec*-butoxy, and *n*-pentoxy.

"Acyl" refers to a group -C(=O)R, where R is hydrogen, alkyl, cycloalkyl, cycloheteroalkyl, aryl, arylalkyl, heteroalkyl, heteroaryl or heteroarylalkyl as defined herein, each of which may be optionally substituted, as defined herein. Representative examples include, but are not limited to formyl, acetyl, cylcohexylcarbonyl, cyclohexylmethylcarbonyl, benzoyl, benzyloxycarbonyl and the like.

"Alkylene" refers to a diradical of a branched or unbranched saturated hydrocarbon chain, having 1-10 carbon atoms, more preferably 1, 2, 3, or 4 carbon atoms. This term is exemplified by groups such as methylene (-CH₂-), ethylene (-CH₂CH₂-), the propylene isomers (e.g., -CH₂CH₂CH₂- and-CH(CH₃)CH₂-) and the like.

"Amino" refers to the group -NR^{y}R^{z} wherein R^{y} and R^{z} are independently selected from the group consisting of hydrogen, alkyl, aryl, heteroaryl (each of which may be optionally substituted), and where R^{y} and R^{z} are optionally joined together with the nitrogen bound thereto to form an optionally substituted heterocycloalkyl.

"Amidino" refers to the group -C(=NR^{x})NR^{y}R^{z} where R^{x}, R^{y}, and R^{z} are independently selected from the group consisting of hydrogen, alkyl, aryl, heteroaryl (each of which may be optionally substituted), and where R^{y} and R^{z} are optionally joined together with the nitrogen bound thereto to form an optionally substituted heterocycloalkyl.

"Amido" refers to both C-amido and N-amido, wherein C-amido refers to the group - C(=O)NR^{y}R^{z} wherein R^{y} and R^{z} are as defined for the amino group, and wherein N-amido refers to the group -N(R^{x})C(=O)R^{y} wherein R^{x} and R^{y} are independently selected from the group consisting of hydrogen, alkyl, aryl, heteroaryl (each of which may be optionally substituted).

"Aryl" refers to a group with one or more aromatic rings. It may be a single aromatic ring or multiple aromatic rings which are fused together, linked covalently, or linked via one or more such as a methylene or ethylene moiety. Aryl groups include, but are not limited to, those groups derived from acenaphthylene, anthracene, azulene, benzene, biphenyl, chrysene, cyclopentadienyl anion, diphenylmethyl, fluoranthene, fluorene, indane, indene, naphthalene, perylene, phenalene, phenanthrene, pyrene, triphenylene, and the like. An aryl group comprises from 5 to about 20 carbon atoms, e.g., from 6 to 20 carbon atoms, e.g. from 6 to 10 carbon atoms.

"Aryloxy" refers to the group -O-aryl, including by way of example, phenoxy and naphthoxy.

"Alkoxycarbonyl" refers to the grpoup -C(O)alkoxy, wherein alkoxy is as defined herein.

"Azido" refers to the group -N₃.

"Carbonyl" refers to the divalent group -C(O)- which is equivalent to -C(=O)-.

"Carboxyl" or "carboxy" refers to -COOH or salts thereof.

"Carboxylate" refers to the group -C(=O)OR^{z} where R^{z} is hydrogen, alkyl, aryl, heteroaryl (each of which may be optionally substituted).

"Carboxyl ester" or "carboxy ester" refers to the groups -C(O)O-alkyl, -C(O)O-substituted alkyl, -C(O)O-alkenyl, -C(O)O-substituted alkenyl, -C(O)O-alkynyl, -C(O)O-substituted alkynyl, -C(O)O-aryl, -C(O)O-substituted aryl, -C(O)O-cycloalkyl, -C(O)O-substituted cycloalkyl, -C(O)O-heteroaryl, -C(O)O-substituted heteroaryl, -C(O)O-heterocyclic, and -C(O)O-substituted heterocyclic wherein alkyl, substituted alkyl, alkenyl, substituted alkenyl, alkynyl, substituted alkynyl, cycloalkyl, substituted cycloalkyl, aryl, substituted aryl, heteroaryl, substituted heteroaryl, heterocyclic, and substituted heterocyclic are as defined herein.

"Cyano" or "carbonitrile" refers to the group -CN.

"Cycloalkyl" refers to a saturated or partially saturated cyclic group of from 3 to about 10 carbon atoms and no ring heteroatoms and having a single ring or multiple rings including fused, bridged, and spiro ring systems. For multiple ring systems having aromatic and non-aromatic rings that have no ring heteroatoms, the term "cycloalkyl" applies when the point of attachment is at a non-aromatic carbon atom (e.g., 5,6,7,8,-tetrahydronaphthalene-5-yl). The term "cycloalkyl" includes cycloalkenyl groups. Examples of cycloalkyl groups include, for instance, adamantyl, cyclopropyl, cyclobutyl, cyclopentyl, cyclooctyl, and cyclohexenyl.

"Halo" or "halogen" refers to fluoro, chloro, bromo and iodo.

"Heteroaryl" refers to an aryl group in which one or more of the carbon atoms (and any associated hydrogen atoms) are each independently replaced with the same or different heteroatoms. Heteroaryl group comprises from 5 to 10 carbon atoms in which 1 to 3 carbon atoms are replaced with heteroatoms that include, but are not limited to, N, P, O, S, etc. Heteroaryl groups include, but are not limited to, groups derived from acridine, carbazole, carboline, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, and the like. A heteroaryl group comprises from 5 to about 20 atoms, e.g., from 5 to 20 atoms, e.g. from 5 to 10 atoms.

"Heterocyclic" refers to a saturated or unsaturated cycloalkyl group in which one or more carbon atoms (and any associated hydrogen atoms) are independently replaced with the same or different heteroatoms. The heterocyclic group comprises from 5 to 10 carbon atoms in which 1 to 3 carbon atoms are replaced with heteroatoms that include, but are not limited to, N, P, O, S, etc. A heterocyclic group may also contain a charged heteroatom or group, e.g., a quatemized ammonium group such as -N⁺(R)₂- wherein R is alkyl, e.g., methyl, ethyl, etc. Heterocyclic groups include, but are not limited to imidazolidine, morpholine, piperazine, piperidine, pyrazolidine, piperidine, pyrrolidine, quinuclidine, and the like.

The terms "optional" or "optionally" mean that the subsequently described event or circumstance may but need not occur, and that the description includes instances where the event or circumstance occurs and instances in which it does not.

"Hydroxy" refers to the group -OH.

"Nitro" refers to the group -NO₂.

"Oxide" refers to products resulting from the oxidation of one or more heteroatoms. Examples include N-oxides, sulfoxides, and sulfones.

"Oxo" refers to the atom (=O).

"Spirocycloalkyl" refers to a 3- to 10- member cyclic substituent formed by replacement of two hydrogen atoms at a common carbon atom with an alkylene group having 2 to 9 carbon atoms, as exemplified by the following structure wherein the methylene group shown below attached to bonds marked with wavy lines is substituted with a spirocycloalkyl group:

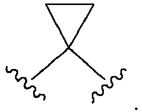

"Stereoisomer" or "stereoisomers" refer to compounds that differ in the chirality of one or more stereocenters. Stereoisomers include enantiomers and diastereomers. The compounds may exist in stereoisomeric form if they possess one or more asymmetric centers or a double bond with asymmetric substitution and, therefore, can be produced as individual stereoisomers or as mixtures. Unless otherwise indicated, the description is intended to include individual stereoisomers as well as mixtures. The methods for the determination of stereochemistry and the separation of stereoisomers are well-known in the art *(see* discussion in Chapter 4 of Advanced Organic Chemistry, 4th ed., J. March, John Wiley and Sons, New York, 1992).

"Substituted" (as in, e.g., "substituted alkyl") refers to a group wherein one or more hydrogens have been independently replaced with one or more substituents including, but not limited to, alkyl, alkenyl, alkynyl, alkoxy, alkoxycarbonyl, acyl, amino, amido, amidino, aryl, azido, carboxyl, carboxyl ester, cyano, cycloalkyl, halo, heteroaryl, heterocyclic, hydroxy, oxo, nitro, sulfonamide, sulfonic acid, thiocyanate, thiol, thione, or a combination thereof. Polymers arrived at by defining substituents with further substituents to themselves ad infinitum (e.g., substituted aryl having a substituted aryl group as a substituent which is itself substituted with a substituted aryl group, which is further substituted by a substituted aryl group, etc.) are not intended for inclusion herein. Unless otherwise noted, the maximum number of such substitutions in compounds described herein is three. For example, serial substitutions of substituted aryl groups with two other substituted aryl groups are limited to -substituted aryl-(substituted aryl)-substituted aryl. Similarly, it is understood that the above definitions are not intended to include impermissible substitution patterns (e.g., methyl substituted with 5 fluoro groups or heteroaryl groups having two adjacent oxygen ring atoms). Such impermissible substitution patterns are well known to the skilled artisan.

"Sulfonamide" and "sulfonamido" refer to S-sulfonamide (or S-sulfonamido) and N-sulfonamide (or N-sulfonamido), wherein S-sulfonamide refers to the group -S(=O)₂NR^{y}R^{z} where R^{y} and R^{z} are as defined for the amino group, and wherein N-sulfonamide refers to the group -N(R^{x})S(=O)₂R^{y} wherein R^{x} and R^{y} are independently selected from the group consisting of hydrogen, alkyl, aryl, heteroaryl (each of which may be optionally substituted).

"Sulfonyl" refers to the group -S(=O)₂R^{x} where R^{x} is selected from the group consisting of hydrogen, alkyl, aryl, heteroaryl, each of which may be optionally substituted.

"Sulfonic acid" refers to the group -SO₃H.

"Thiocyanate" refers to the group -SCN.

"Thiol" refers to the group -SH.

"Thione" refers to the atom (=S).

"Pharmaceutically acceptable" refers to that which is useful in preparing a pharmaceutical composition that is generally safe, non-toxic, and neither biologically nor otherwise undesirable, and includes that which is acceptable for veterinary or human pharmaceutical use.

"Pharmaceutically acceptable salt" refers to a salt of a compound that is pharmaceutically acceptable and that possesses (or can be converted to a form that possesses) the desired pharmacological activity of the parent compound. Such salts include acid addition salts formed with inorganic acids such as hydrochloric acid, hydrobromic acid, sulfuric acid, nitric acid, phosphoric acid, and the like; or formed with organic acids such as acetic acid, benzenesulfonic acid, benzoic acid, camphorsulfonic acid, citric acid, ethanesulfonic acid, fumaric acid, glucoheptonic acid, gluconic acid, lactic acid, maleic acid, malonic acid, mandelic acid, methanesulfonic acid, 2-napththalenesulfonic acid, oleic acid, palmitic acid, propionic acid, stearic acid, succinic acid, tartaric acid, p-toluenesulfonic acid, trimethylacetic acid, and the like, and salts formed when an acidic proton present in the parent compound is replaced by either a metal ion, e.g., an alkali metal ion, an alkaline earth ion, or an aluminum ion; or coordinates with an organic base such as diethanolamine, triethanolamine, *N*-methylglucamine and the like. Also included in this definition are ammonium and substituted or quaternized ammonium salts. Representative non-limiting lists of pharmaceutically acceptable salts can be found in S.M. Berge et al., J. Pharma Sci., 66(1), 1-19 (1977), and Remington: The Science and Practice of Pharmacy, R. Hendrickson, ed., 21st edition, Lippincott, Williams & Wilkins, Philadelphia, PA, (2005), at p. 732, Table 38-5, each of which are hereby incorporated by reference herein.

### Compounds

In one embodiment, this invention provides a compound of formula I: 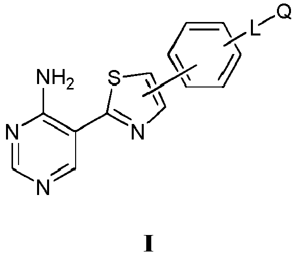 wherein:
L is selected from the group consisting of -NHC(X)-, -C(X)NH-, -NHC(X)NH-, -C(X)-, -C(X)NH-alkylene-, and -NHC(X)-alkylene-, where X is oxygen or sulfur; and
Q is selected from the group consisting of alkyl, substituted alkyl, aryl, substituted aryl, heteroaryl, substituted heteroaryl, cycloalkyl, substituted cycloalkyl, heterocyclic, and substituted heterocyclic,
or a pharmaceutically acceptable salt thereof.

The compounds are VEGFR2 kinase inhibitors, and are useful in the treatment of diseases capable of treatment by a VEGFR2 kinase inhibitor, particularly cancer. In another embodiment, this invention provides pharmaceutical compositions comprising the compounds of formula I for use as VEGFR2 kinase inhibitors and for use in the treatment of diseases capable of treatment by a VEGFR2 kinase inhibitor, particularly cancer. In another embodiment, this invention provides methods of treatment of diseases capable of treatment by a VEGFR2 kinase inhibitor, particularly cancer, by administration of the compounds. In another embodiment, this invention provides the use of the compounds in the manufacture of medicaments for use as a VEGFR2 kinase inhibitor and for use in the treatment of diseases capable of treatment by a VEGFR2 kinase inhibitor, particularly cancer.

In another embodiment, this invention provides a compound represented by formula IA: 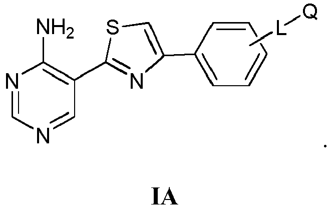 wherein:
L is selected from the group consisting of -NHC(X)-, -C(X)NH-, -NHC(X)NH-, -C(X)-, -C(X)NH-alkylene-, and -NHC(X)-alkylene-, where X is oxygen or sulfur; and
Q is selected from the group consisting of alkyl, substituted alkyl, aryl, substituted aryl, heteroaryl, substituted heteroaryl, cycloalkyl, substituted cycloalkyl, heterocyclic, and substituted heterocyclic,
or a pharmaceutically acceptable salt thereof.
In some embodiments, Q is selected from the group consisting of aryl, substituted aryl, heteroaryl, and substituted heteroaryl. In some embodiments, Q is selected from the group consisting of phenyl, substituted phenyl, pyridyl, and substituted pyridyl.

In another embodiment, this invention provides a compound represented by formula IB: 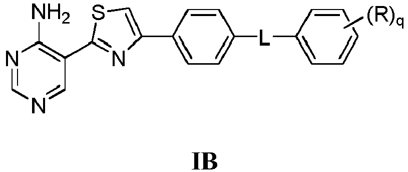 wherein:
L is selected from the group consisting of -NHC(X)-, -C(X)NH-, -NHC(X)NH-, -C(X)-, -C(X)NH-alkylene-, and -NHC(X)-alkylene-, where X is oxygen or sulfur;
q is 0, 1, or 2; and
each R is independently selected from the group consisting of halo, alkyl, substituted alkyl, alkoxy, amino, cyano, nitro, heteroaryl, substituted heteroaryl, carboxy, and alkxoxycarbonyl; or
when q is 2, the two R can join to form a ring;
or a pharmaceutically acceptable salt thereof.

In some embodiments, L is selected from the group consisting of -NHC(X)- and - NHC(X)NH-. In some embodiments, X is O. In some embodiments, q is 1 or 2. In some embodiments, each R is independently selected from the group consisting of halo, alkyl, and substituted alkyl.

In another embodiment, this invention provides a compound represented by formula IC: 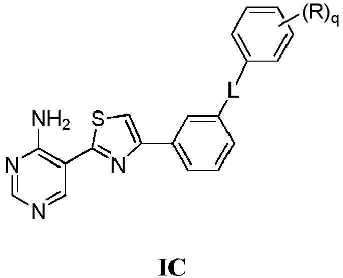 wherein:
L is selected from the group consisting of -NHC(X)-, -C(X)NH-, -NHC(X)NH-, -C(X)-, -C(X)NH-alkylene-, and -NHC(X)-alkylene-, where X is oxygen or sulfur;
q is 0, 1, or 2;
each R is independently selected from the group consisting of halo, alkyl, substituted alkyl, alkoxy, amino, cyano, nitro, heteroaryl, substituted heteroaryl, carboxy, and alkxoxycarbonyl; or
when q is 2, the two R can join to form a ring,
or a pharmaceutically acceptable salt thereof.

In some embodiments, L is selected from the group consisting of -NHC(X)-, -C(X)NH-, -NHC(X)NH-, -C(X)NH-alkylene-, and -NHC(X)-alkylene-. In some embodiments, alkylene is CH₂. In some embodiments, X is O. In some embodiments, q is 1 or 2.

In another embodiment, this invention provides a compound is represented by formula ID: 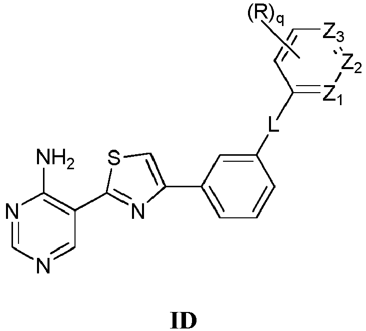 wherein:
q is 0, 1, or 2;
L is selected from the group consisting of -NHC(X)-, -C(X)NH-, -NHC(X)NH-, -C(X)-, -C(X)NH-alkylene-, and -NHC(X)-alkylene-, where X is oxygen or sulfur;
each R is independently selected from the group consisting of halo, alkyl, substituted alkyl, alkoxy, amino, cyano, nitro, heteroaryl, substituted heteroaryl, carboxy, and alkxoxycarbonyl; or
when q is 2, the two R can join to form a ring; and
each of Z₁, Z₂, and Z₃ is independently N or CH,
or a pharmaceutically acceptable salt thereof.

In some embodiments, L is -NHC(X)-. In some embodiments, X is O. In some embodiments, q is 0 or 1. In some embodiments, R is alkyl. In some embodiments, L is selected from the group consisting of NHC(X)-, -NHC(X)NH-, -C(X)-, and -NHC(X)-alkylene-, where X is oxygen or sulfur. In some embodiments, alkylene is CH₂. In some embodiments, X is O. In some embodiments, Q is selected from the group consisting of alkyl, substituted alkyl, cycloalkyl, substituted cycloalkyl, heterocyclic, and substituted heterocyclic.

In another embodiment, this invention provides a compound of formula II: 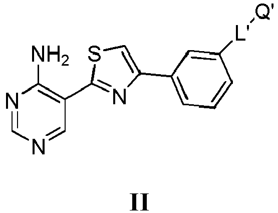 wherein:
L' is selected from the group consisting of -NHC(X)- and -NHC(X)NH-; and
Q' is selected from the group consisting of aryl, substituted aryl, cycloalkyl, and substituted cycloalkyl,
or a pharmaceutically acceptable salt thereof.

In some embodiments, X is O. In some embodiments, Q' is selected from the group consisting of phenyl substituted phenyl, cycloalkyl, and substituted cycloalkyl. In some embodiments, the compound is represented by formula IIA: 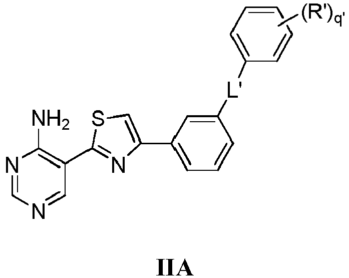 wherein:
L' is selected from the group consisting of -NHC(X)- and -NHC(X)NH-;
q' is 1 or 2;
each R' is independently selected from the group consisting of halo, alkyl, substituted alkyl, alkoxy, amino, cyano, nitro, heteroaryl, substituted heteroaryl, carboxy, and alkxoxycarbonyl; or
when q' is 2, the two R' can join to form a ring,
or a pharmaceutically acceptable salt thereof.

In another embodiment, this invention provides a compound represented by formula IIB: 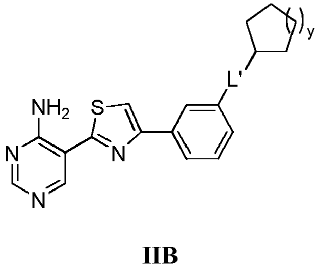 wherein:
L' is selected from the group consisting of -NHC(X)- and -NHC(X)NH-; and
y is 1, 2, or 3 .

Representative examples of compounds of formula I include the compounds listed in Tables 1-4 below. In another embodiment, this invention provides a compound selected from the compounds listed in Tables 1-4.

**Table 1**

| | | |
|---|---|---|
| 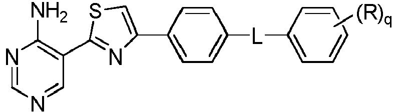 | | |

| **Cmpd #** | **L** | **(R)q** |
|---|---|---|
| 1 | NHC(O)NH | 3-CF₃ |
| 2 | NHC(O)NH | 3-CF₃-4-F |

**Table 2**

| | | | | | |
|---|---|---|---|---|---|
| 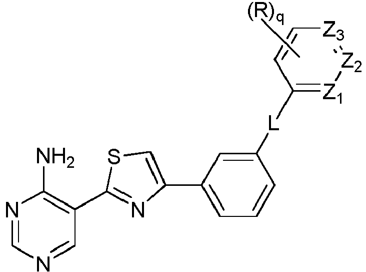 | | | | | |

| **Cmpd #** | **L** | **(R)q** | **Z₁** | **Z₂** | **Z₃** |
|---|---|---|---|---|---|
| 3 | NHC(O) | H | CH | CH | CH |
| 4 | NHC(O)NH | H | CH | CH | CH |
| 5 | NHC(O) | 4-F | CH | CH | CH |
| 6 | NHC(O) | 3-Cl | CH | CH | CH |
| 7 | NHC(O) | 3,4-di-Cl | CH | CH | CH |
| 8 | NHC(O) | 4-Cl | CH | CH | CH |
| 9 | NHC(O) | 3,4-di-F | CH | CH | CH |
| 10 | NHC(O)NH | 3-CF₃-4-F | CH | CH | CH |
| 11 | NHC(O)NH | 3-Cl-4-Me | CH | CH | CH |
| 12 | NHC(O)NH | 3-F | CH | CH | CH |
| 13 | NHC(O) | 4-NO₂ | CH | CH | CH |
| 14 | NHC(O)NH | 4-OMe | CH | CH | CH |
| 15 | NHC(O) | 4-Ph | CH | CH | CH |
| 16 | NHC(O)NH | 4-Cl | CH | CH | CH |
| 17 | NHC(O)NH | 3-Me | CH | CH | CH |
| 18 | NHC(O)NH | 2-Cl | CH | CH | CH |
| 19 | NHC(O)NH | 2-Me | CH | CH | CH |
| 20 | NHC(O)NH | 2-CF₃ | CH | CH | CH |
| 21 | NHC(O) | H | N | CH | CH |
| 22 | NHC(O)NH | 3-CF₃-4-Cl | CH | CH | CH |
| 23 | NHC(O) | H | CH | N | CH |
| 24 | NHC(O) | H | CH | CH | N |
| 25 | NHC(O) | 2-OMe | CH | CH | CH |
| 26 | NHC(O)NH | 2-OMe | CH | CH | CH |
| 27 | NHC(O)NH | 3,4-di-Me | CH | CH | CH |
| 28 | NHC(O)NH | 4-Et | CH | CH | CH |
| 29 | NHC(O)NH | 4-CN | CH | CH | CH |
| 30 | NHC(O)NH | 3-CN | CH | CH | CH |
| 31 | NHC(O)NH | 3-Cl | CH | CH | CH |
| 32 | NHC(O)NH | 3,5-di-Cl | CH | CH | CH |
| 33 | NHC(O)NH | 2-NO₂ | CH | CH | CH |
| 34 | NHC(O)NH | 4-NO₂ | CH | CH | CH |
| 35 | NHC(O)NH | 4-NH₂ | CH | CH | CH |
| 36 | NHC(O)NH | 2-C(O)OBu | CH | CH | CH |
| 37 | NHC(O) | 4-Me | CH | N | CH |
| 38 | NHC(O)NH | 2-Me-6-Et | CH | CH | CH |
| 39 | NHC(O)NH | 3-CF₃ | CH | CH | CH |
| 40 | NHC(O)CH₂ | 3-CF₃ | CH | CH | CH |
| 41 | NHC(O) | 3-Me | CH | CH | CH |
| 42 | NHC(O)NH | 3-C(O)OEt | CH | CH | CH |
| 43 | NHC(O)NH | 3-C(Me)₂OH | CH | CH | CH |
| 44 | NHC(O)NH | 3-CH₂OH | CH | CH | CH |
| 45 | NHC(O)CH₂ | H | CH | CH | CH |
| 46 | NHC(O)NH | 3-morpholinomethyl | CH | CH | CH |
| 47 | NHC(O)NH | 3-(5-methyl-1,2,4- | CH | CH | CH |
| 48 | C(O)NH | H | CH | CH | CH |
| 49 | C(O)NH | 3-Me | CH | CH | CH |
| 50 | C(O)NHCH₂ | H | CH | CH | CH |
| 51 | C(O)NHCH₂ | 4-Me | CH | CH | CH |
| 52 | NHC(O) | 3-morpholinomethyl | CH | CH | CH |
| 53 | NHC(O) | 3-(piperidin-1- | CH | CH | CH |
| 54 | NHC(O)NH | 3-C(O)OH | CH | CH | CH |
| 55 | NHC(O) | 3-CH₂NMe₂ | CH | CH | CH |
| 56 | NHC(O) | 3-CH₂NH(CH₂)₂OH | CH | CH | CH |
| 57 | NHC(O)NH | 3-CH₂OC(O)Me | CH | CH | CH |

**Table 3**

| | | |
|---|---|---|
| 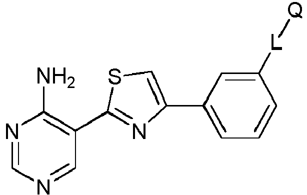 | | |

| **Cmpd #** | **L** | **Q** |
|---|---|---|
| 58 | NHC(O)NH | CH₂Ph |
| 59 | NHC(O)NH | CH₂C(O)OEt |
| 60 | NHC(O)NH | Et |
| 61 | NHC(O) | Me |
| 62 | NHC(O) | cyclohexyl |
| 63 | NHC(O)NH | cyclopentyl |
| 64 | NHC(O) | cyclopentyl |
| 65 | NHC(O)NH | cyclohexyl |
| 66 | NHC(O)CH₂ | morpholin-1-yl |
| 67 | NHC(O)CH₂ | piperidin-1-yl |
| 68 | NHC(O) | piperidin-1-yl |
| 69 | NHC(O) | 4-hydroxypiperidin-1-yl |
| 70 | NHC(O) | 3-hydroxypiperidin-1-yl |
| 71 | C(O) | morpholin-1-yl |

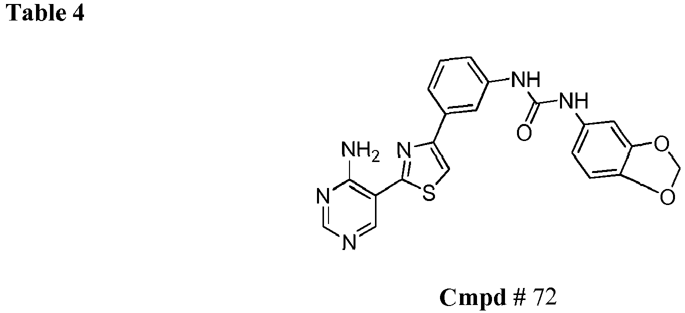

### Pharmaceutical Compositions and Administration

Compounds provided in accordance with the present disclosure are usually administered in the form of pharmaceutical compositions. This disclosure therefore provides pharmaceutical compositions that contain, as the active ingredient, one or more of the compounds described, or a pharmaceutically acceptable salt or ester thereof, and one or more pharmaceutically acceptable excipients, carriers, including inert solid diluents and fillers, diluents, including sterile aqueous solution and various organic solvents, permeation enhancers, solubilizers and adjuvants. The pharmaceutical compositions may be administered alone or in combination with other therapeutic agents. Such compositions are prepared in a manner well known in the pharmaceutical art (see, e.g., Remington's Pharmaceutical Sciences, Mace Publishing Co., Philadelphia, PA 17th Ed. (1985); and Modem Pharmaceutics, Marcel Dekker, Inc. 3rd Ed. (G.S. Banker & C.T. Rhodes, Eds.)

In another embodiment, this invention provides a pharmaceutical composition comprising a compound as described herein and a pharmaceutically acceptable excipient.

The compounds of this invention may be administered by any route suitable to the subject being treated and the nature of the subject's condition. Routes of administration include administration by injection, including intravenous, intraperitoneal, intramuscular, and subcutaneous injection, by transmucosal or transdermal delivery, through topical applications, nasal spray, suppository and the like or may be administered orally. Formulations may optionally be liposomal formulations, emulsions, formulations designed to administer the drug across mucosal membranes or transdermal formulations. Suitable formulations for each of these methods of administration may be found, for example, in Remington: The Science and Practice of pharmacy, 20th ed., A. Gennaro, ed., Lippincott Williams & Wilkins, Philadelphia, Pennsylvania, U.S.A. Typical formulations will be either oral or solutions for intravenous infusion. Typical dosage forms will be tablets or capsules for oral administration, solutions for intravenous infusion, and lyophilized powders for reconstitution as solutions for intravenous infusion.

Depending on the intended mode of administration, the pharmaceutical compositions may be in the form of solid, semi-solid or liquid dosage forms, preferably in unit dosage form suitable for single administration of a precise dosage. In addition to an effective amount of the active compound(s), the compositions may contain suitable pharmaceutically-acceptable excipients, including adjuvants which facilitate processing of the active compounds into preparations which can be used pharmaceutically. "Pharmaceutically acceptable excipient" refers to an excipient or mixture of excipients which does not interfere with the effectiveness of the biological activity of the active compound(s) and which is not toxic or otherwise undesirable to the subject to which it is administered.

For solid compositions, conventional excipients include, for example, pharmaceutical grades of mannitol, lactose, starch, magnesium stearate, sodium saccharin, talc, cellulose, glucose, sucrose, magnesium carbonate, and the like. Liquid pharmacologically administrable compositions can, for example, be prepared by dissolving, dispersing, etc., an active compound as described herein and optional pharmaceutical adjuvants in water or an aqueous excipient, such as, for example, water, saline, aqueous dextrose, and the like, to form a solution or suspension. If desired, the pharmaceutical composition to be administered may also contain minor amounts of nontoxic auxiliary excipients such as wetting or emulsifying agents, pH buffering agents and the like, for example, sodium acetate, sorbitan monolaurate, triethanolamine sodium acetate, triethanolamine oleate, etc.

For oral administration, the composition will generally take the form of a tablet or capsule, or it may be an aqueous or nonaqueous solution, suspension or syrup. Tablets and capsules are preferred oral administration forms. Tablets and capsules for oral use will generally include one or more commonly used excipients such as lactose and corn starch. Lubricating agents, such as magnesium stearate, are also typically added. When liquid suspensions are used, the active agent may be combined with emulsifying and suspending excipients. If desired, flavoring, coloring and/or sweetening agents may be added as well. Other optional excipients for incorporation into an oral formulation include preservatives, suspending agents, thickening agents, and the like.

Injectable formulations can be prepared in conventional forms, either as liquid solutions or suspensions, solid forms suitable for solubilization or suspension in liquid prior to injection, or as emulsions or liposomal formulations. The sterile injectable formulation may also be a sterile injectable solution or a suspension in a nontoxic parenterally acceptable diluent or solvent. Among the acceptable vehicles and solvents that may be employed are water, Ringer's solution and isotonic sodium chloride solution. In addition, sterile, fixed oils, fatty esters or polyols are conventionally employed as solvents or suspending media.

The pharmaceutical compositions of this invention may also be formulated in lyophilized form for parenteral administration. Lyophilized formulations may be reconstituted by addition of water or other aqueous medium and then further diluted with a suitable diluent prior to use. The liquid formulation is generally a buffered, isotonic, aqueous solution. Examples of suitable diluents are isotonic saline solution, 5% dextrose in water, and buffered sodium or ammonium acetate solution. Pharmaceutically acceptable solid or liquid excipients may be added to enhance or stabilize the composition, or to facilitate preparation of the composition.

Typically, a pharmaceutical composition of the present invention is packaged in a container with a label, or instructions, or both, indicating use of the pharmaceutical composition in the treatment of a disease treatable by administration of a VEGFR2 kinase inhibitor, particularly cancer.

The pharmaceutical composition may additionally contain one or more other pharmacologically active agents in addition to a compound of this invention. These additional active agents will typically be useful in treating cancer, or for enhancing the treatment of cancer by compounds of this invention.

### Synthesis of Compounds

The compounds of the disclosure may be prepared using methods disclosed herein and routine modifications thereof which will be apparent given the disclosure herein and methods well known in the art. Conventional and well-known synthetic methods may be used in addition to the teachings herein. The synthesis of compounds described herein, e.g. compounds having structures described by one or more of Formula I, may be accomplished as described in the following examples. If available, reagents may be purchased commercially, e.g. from Sigma Aldrich or other chemical suppliers.

### General Syntheses

Embodiments of compounds in accordance with the present disclosure may be synthesized using the general reaction schemes described below. It will be apparent given the description herein that the general schemes may be altered by substitution of the starting materials with other materials having similar structures to result in products that are correspondingly different. Descriptions of syntheses follow to provide numerous examples of how the starting materials may vary to provide corresponding products. Given a desired product for which the substituent groups are defined, the necessary starting materials generally may be determined by inspection. Starting materials are either obtained from commercial sources or synthesized using published methods. For synthesizing compounds which are embodiments of the present disclosure, inspection of the structure of the compound to be synthesized will provide the identity of each substituent group. The identity of the final product will generally render apparent the identity of the necessary starting materials by a simple process of inspection, given the examples herein.

The compounds of this disclosure can be prepared from readily available starting materials using, for example, the following general methods and procedures. It will be appreciated that where typical process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. Suitable protecting groups for various functional groups as well as suitable conditions for protecting and deprotecting particular functional groups are well known in the art. For example, numerous protecting groups are described in T. W. Greene and G. M. Wuts (1999) Protecting Groups in Organic Synthesis, 3rd Edition, Wiley, New York, and references cited therein.

Furthermore, the compounds of this disclosure may contain one or more chiral centers. Accordingly, if desired, such compounds can be prepared or isolated as pure stereoisomers, i.e., as individual enantiomers or diastereomers, or as stereoisomer-enriched mixtures. All such stereoisomers (and enriched mixtures) are included within the scope of this disclosure, unless otherwise indicated. Pure stereoisomers (or enriched mixtures) may be prepared using, for example, optically active starting materials or stereoselective reagents well-known in the art. Alternatively, racemic mixtures of such compounds can be separated using, for example, chiral column chromatography, chiral resolving agents, and the like.

The starting materials for the following reactions are generally known compounds or can be prepared by known procedures or obvious modifications thereof. For example, many of the starting materials are available from commercial suppliers such as Aldrich Chemical Co. (Milwaukee, Wisconsin, USA), Bachem (Torrance, California, USA), Emka-Chemce or Sigma (St. Louis, Missouri, USA). Others may be prepared by procedures, or obvious modifications thereof, described in standard reference texts such as Fieser and Fieser's Reagents for Organic Synthesis, Volumes 1-15 (John Wiley, and Sons, 1991), Rodd's Chemistry of Carbon Compounds, Volumes 1-5, and Supplementals (Elsevier Science Publishers, 1989), Organic Reactions, Volumes 1-40 (John Wiley, and Sons, 1991), March's Advanced Organic Chemistry, (John Wiley, and Sons, 5th Edition, 2001), and Larock's Comprehensive Organic Transformations (VCH Publishers Inc., 1989).

### Synthesis of the Compounds of Formula I

In one embodiment, the compounds of Formula I are prepared by using the general method described in Scheme I below. Starting with 4-aminopyrimidine-5-carbothioamide (A), which may be commercially obtained, or synthesized *de novo,* and then adding 2-bromo-1-(nitrophenyl)ethanone (B), the 2,4-disubstituted thiazole (C) is obtained. Reduction of the nitro group in C with hydrogen using as Pd/C catalyst provides the amino derivative D. Coupling of D with an isocyanate in the presence of a base such as triethylamine, provides compounds of formula I, where L = -NHC(O)NH-. Similarly, Coupling of D with an acyl halide, such as an acyl chloride, in the presence of a base such as triethylamine, provides compounds of formula I, where L = -NHC(O)-.

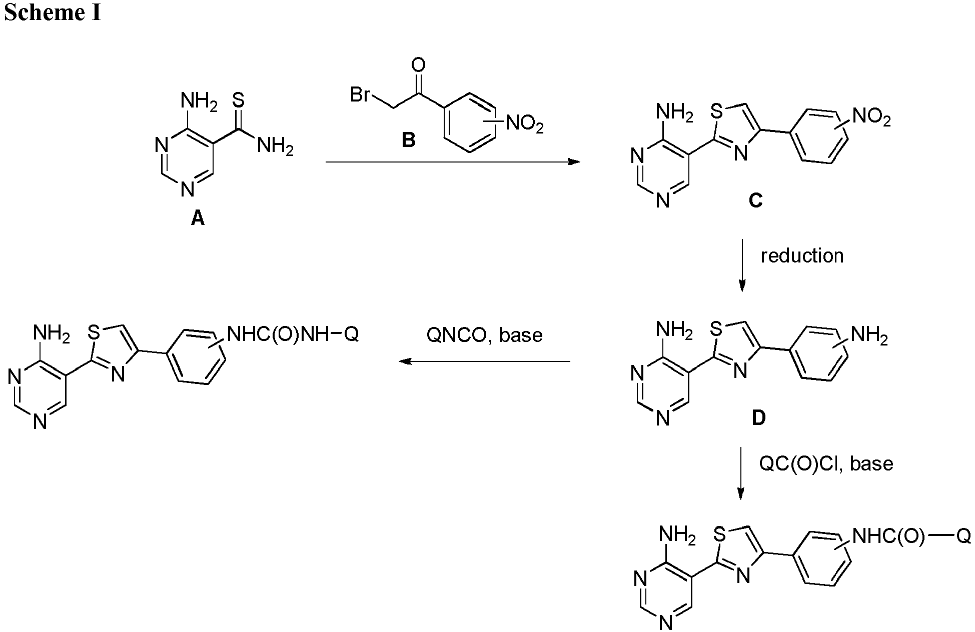

Compounds of formula I may be converted to salts by reaction with the appropriate acids, using techniques well known to a person of ordinary skill in the art for the formation of acid addition salts. The acid used, and the reaction conditions, may be chosen to give salts that are pharmaceutically acceptable and that have a form convenient for isolation and formulation, such as a solid form (for example, amorphous or crystalline).

### Methods

The compounds of this invention have activity against human cancer cell lines, as demonstrated in the *in vitro* and *in vivo* Examples below, and are therefore considered to be useful as human cancer chemotherapeutic agents, for the treatment of human cancers.

Thus, this invention includes methods of treating cancer in humans by administering a therapeutically effective amount of a compound of this invention, or a pharmaceutical composition containing it, to the human; and to the use of the compounds of this invention in the manufacture of medicaments for the treatment of cancer in humans. Optionally, the methods further comprise treating the human with another anticancer therapy, such as a therapy already conventional for the cancer being treated.

Cancers that are particularly treatable by the method of this invention are cancers that are treatable using VEGFR2 (vascular endothelial growth factor receptor 2) kinase inhibitors. Such cancers include hematological malignancies, such as leukemias, lymphomas, and myelodysplastic syndrome. Other cancers particularly treatable by the method of this invention include solid malignancies such as colorectal, lung, breast, ovarian, pancreatic, bladder, brain, gastrointestinal, and kidney cancers, and hematological malignancies, such as leukemias, especially ALL and CML, lymphomas, and myelodysplastic syndrome.

The amount of the compound of this invention that is administered to the human (either alone or, more usually, in a composition of this invention) is a therapeutically effective amount when used alone or when used in conjunction with the another anticancer therapy (if the compound of this invention is administered in conjunction with another anticancer therapy); and similarly the amount of the another anticancer therapy that is administered to the human (if the compound of this invention is administered in conjunction with another anticancer therapy) is a therapeutically effective amount when used in conjunction with the compound of this invention. However, the therapeutically effective amount of either the compound of this invention and the amount of the another anticancer therapy when administered in combination cancer chemotherapy may each be less than the amount which would be therapeutically effective if delivered to the human alone. It is common in cancer therapy, though, to use the maximum tolerated dose of the or each therapy, with a reduction only because of common toxicity of the therapies used or potentiation of the toxicity of one therapy by another.

The compounds of this invention, or pharmaceutical compositions containing them, are thus used to treat cancer in humans requiring such treatment, by administering a therapeutically effective amount of the chosen compound or composition. Therapeutically effective amounts of compounds of the invention are in the range of 10-10,000 mg/m², for example, 30-3000 mg/m² or 100-1000 mg/m². Dosing may be at 1-35 day intervals; for example, about 500-1000 mg/m² at 1-5 week intervals, especially at 1, 2, 3, or 4 week intervals, or at higher frequencies including as frequently as once/day for several (e.g. 5 or 7) days, with the dosing repeated every 2, 3, or 4 weeks, or constant infusion for a period of 6-72 hours, also with the dosing repeated every 2, 3, or 4 weeks. Suitable dosages and dose frequencies will be readily determinable by a person of ordinary skill in the art having regard to that skill and this disclosure. No unacceptable toxicological effects are expected when compounds of the invention are administered in accordance with the present invention.

A person of ordinary skill in the art of cancer therapy will be able to ascertain a therapeutically effective amount of the compound of this invention and a therapeutically effective amount of another anticancer therapy (if the compound of this invention is administered as a part of a chemotherapeutic combination) for a given cancer and stage of disease without undue experimentation and in reliance upon personal knowledge and the disclosure of this application.

In another embodiment, this invention provides a method of inhibiting VEGFR2 kinase using a compound as described herein. In another embodiment, this invention provides a compound as described herein for use as a VEGFR2 kinase inhibitor or as a VEGFR2 kinase inhibitor in the treatment of cancer

In another embodiment, this invention provides a method of treating a cancer patient in need of an inhibitor of VEGFR2 kinase, comprising: administering an effective amount of a compound as described herein. In a further embodiment, the compound has cytotoxicity against HCT116 of 40 µM or less.

### EXAMPLES

The following examples are included to demonstrate some embodiments of the disclosure. It should be appreciated by those of skill in the art that the techniques disclosed in the examples which follow represent techniques discovered by the inventor to function well in the practice of the disclosure, and thus can be considered to constitute the modes for its practice. However, those of skill in the art should, in light of the present disclosure, appreciate that many changes can be made in the specific embodiments which are disclosed and still obtain a like or similar result without departing from the spirit and scope of the disclosure. The compounds listed in Tables 1-4 were synthesized using the procedures described herein as well as using the methods known to one of skill in the art. As can be seen from Table 5 in example 5, the compounds of this invention were synthesized and tested for the biological activity.

It will also be appreciated that the addition of any substituent may result in the production of a number of isomeric products any or all of which may be isolated and purified using conventional techniques.

Unless otherwise stated, all temperatures are in degrees Celcius (°C) and the following abbreviations have the following definitions:
- DMF: = dimethylformamide
- EtOH: = ethanol
- ETOAc: = Ethyl acetate
- MeOH: = methanol
- µL: = micro liter
- LC: = Liquid chromatography
- MS: = Mass spectrometry
- mL: = mililiter
- TEA: = triethylamine
- THF: = tetrahydrofuran
- µM: = Micromolar
- DMSO: = dimethylsulfoxide
- IV: = intravenous
- w/w: = Weight by weight

### Example 1: Preparation of 1-(3-(2-(4-aminopyrimidin-5-yl)thiazol-4-yl)phenyl)-3-m-tolylurea from 5-(4-(3-aminophenyl)thiazole-2-yl)pyrimidin-4-amine (compound 17)

### Step 1: Preparation of 5-(4-(3-nitrophenyl)thiazol-2-yl)pyrimidin-4-amine

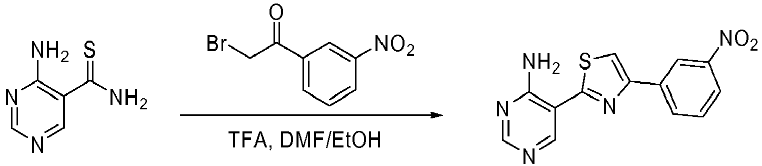

4-aminopyrimidine-5-carbothioamide (2.00 g, 11.6 mmol) was added to a 40 mL scintillation vial and dissolved in DMF/EtOH (1:3, 20 mL). Trifluoroacetic acid (2.4 mL, 32.4 mmol) was then added and the mixture was heated to 80 °C. After the thioamide was dissolved, 2-bromo-1-(3-nitrophenyl)ethanone was added in portions over 10 minutes. The reaction was stirred at 60 °C for about 12 hours. The reaction was cooled to room temperature and the solid (product) was filtered out. The solid was washed with EtOH and EtOAc and the volatiles were removed under reduced pressure to afford 5-(4-(3-nitrophenyl)thiazol-2-yl)pyrimidin-4-amine as a light yellow solid. LC-MS (ES⁺): *m*/*z* = 300 [C₁₃H₉N₅O₂S+H⁺] for product peak.

### Step 2: Preparation of 5-(4-(3-aminophenyl)thiazol-2-yl)pyrimidin-4-amine

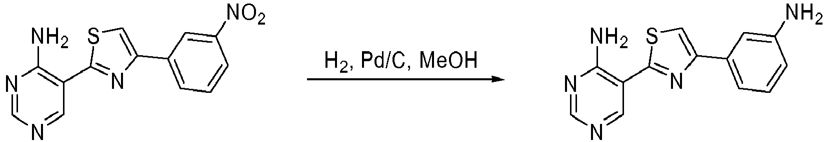

5-(4-(3-nitrophenyl)thiazol-2-yl)pyrimidin-4-amine (2.0g, 6.7 mmol) was suspended in hot MeOH (-150 mL). The mixture was allowed to cool and 10% Pd/C (degussa) (900 mg) was then added and the reaction was agitated with a Parr-shaker for 48 hours under H₂ (65 p.s.i.). The Pd/C was then filtered over Celite (-2 g) and the Celite/Pd/C mixture was washed repeatedly with refluxing MeOH for 48 hours using a Soxhlet extractor. The MeOH solutions were combined and the MeOH was evaporated to give 5-(4-(3-aminophenyl)thiazol-2-yl)pyrimidin-4-amine (1.4 g). LC-MS (ES⁺): *m*/*z* = 270 [C₁₃H₉N₅O₂S+H⁺] for product peak.

### Step 3: Preparation of 1-(3-(2-(4-aminopyrimidin-5-yl)thiazol-4-yl)phenyl)-3-m-tolylurea (compound 17)

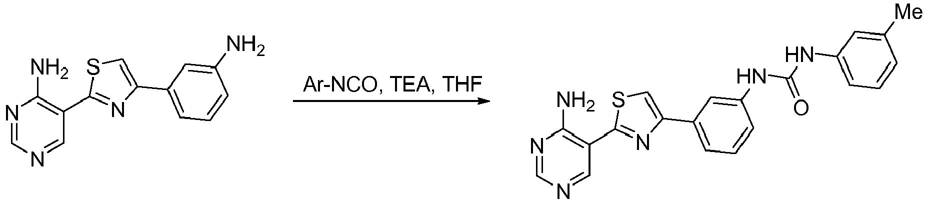

5-(4-(3-aminophenyl)thiazole-2-yl)pyrimidin-4-amine (150 mg, 0.557 mmol) from step 2 was added to a 40 mL scintillation vial, and dissolved with 2 mL of tetrahydrofuran and let stir at room temperature. Triethylamine was added (227 µL, 1.671 mmol), followed by *m*-tolyl isocyanate (74 mg, 0.557 mmols). Stirring was continued at room temperature for two hours. The tetrahydrofuran was removed under reduced pressure, and the residue suspended in water
(5 mL). The solid was filtered and washed with ethyl acetate (2 mL) and dried on the high vacuum overnight to give 1-(3-(2-(4-aminopyrimidin-5-yl)thiazol-4-yl)phenyl)-3-*m*-tolylurea as a yellow solid (150 mg, 67% yield). LC-MS (ES⁺): *m*/*z* = 403.1 [C₂₁H₁₈N₆OS+H⁺] for product peak.

### Example 2: Preparation of N-(3-(2-(4-aminopyrimidin-5-yl)thiazol-4-yl)phenyl)-3-chlorobenzamide from 5-(4-(3-aminophenyl)thiazole-2-yl)pyrimidin-4-amine (compound 6)

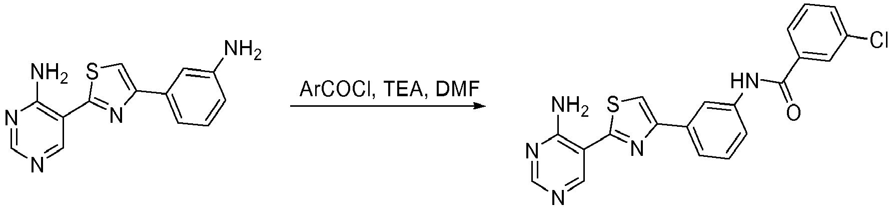

5-(4-(3-aminophenyl)thiazole-2-yl)pyrimidin-4-amine (500mg, 1.858 mmol) from step 2 of example 1 was added to a 40 mL scintillation vial, and dissolved with 1 mL of dimethylformamide and let stir in an ice bath at 0 °C. Triethylamine was added (381 µL, 2.788 mmol), followed by 3-chlorobenzoyl chloride (216 µL, 1.858 mmols). Stirring was continued while the reaction mixture was allowed to warm to room temperature for 2 hours. Water (5 mL) was added to the mixture, the solid was filtered, and dried on the high vacuum overnight to give *N*-(3-(2-(4-aminopyrimidin-5-yl)thiazol-4-yl)phenyl)-3-chlorobenzamide as a yellow solid (150 mg, 20% yield). LC-MS (ES⁺): *m*/*z* = 408 [C₂₀H₁₄ClN₅OS+H⁺] for product peak.

Using the procedure described in Examples 1 and 2 and the general procedure described herein as well as those known to one of skill in the art, the other compounds of the invention were prepared using analogous methods. The mass spectra data for the compounds is shown in Table 5. The ¹H NMR spectra taken for each of the compounds 1-72 were found to be consistent with their structures depicted.

### In vitro examples

The following examples illustrate the cytotoxic / cytostatic effect of the compounds against human cancer cell lines *in vitro.* These results are considered predictive of efficacy in human cancer chemotherapy, as other anticancer agents tested in these assays have shown anticancer activity in humans.

The cell lines HL60 (human promyelocytic leukemia) and HCT116 (human colon carcinoma) were obtained from the American Type Culture Collection, Manassas, Virginia, U.S.A. All products were used in accordance with manufacturer's directions. The cytotoxicity assays were conducted in triplicate, in each case with solvent control.

### Example 3: In vitro VEGFR2 kinase assay

50 ng/well VEGFR2 kinase (from InVitrogen) was mixed in buffer (14.5 µL/well). To this were added, 0.5 µL of the compound of this invention at a concentration of 2.5% in DMSO. After incubatiuon for 5 minutes at room temperature, 5 µL of substrate (CSKtide, 400 nM) and 8 µL ATP were added. The mixture was incubated for 60 minutes at room temperature and 60 uL of binding reagent (beads) was added. After incubation overnight at room temperature, the plate was read with a LJL plate reader. All of the compounds from Tables 1-4 showed VEGFR2 inhibition with an IC₅₀ of 2000 nm or less as shown in Table 5.

### Example 4: In vitro Cytotoxicity assays

Log-phase cells were trypsinized, collected by centrifugation, and resuspended in a small volume of fresh medium, and the density of viable cells was determined following Trypan Blue staining. Cells were diluted in fresh media, the test compounds (concentrations between 0.1 µM and 200 µM, dissolved in DMSO, 50 µL) added immediately after dilution to achieve a final DMSO concentration of 0.5%, then the suspensions added at 150 µL/well to 96-well plates, and incubated overnight to allow attachment in the case of adherent cells. The cells were cultured for three days (about three doubling times). The cells were then collected by centrifugation, and 100 µL of the culture supernatant was replaced by the CellTiter-Glo reagent. After incubation for 15 minutes at room temperature, the plate was read with a luminometer. Selected compounds from Tables 1-4 showed cytotoxicity in HL-60 (human promylocytic leukemia) assays and HCT 116 (human colon carcinoma) assays as shown in Table 5. The table gives cytotoxicity IC₅₀s in µM.

### In vivo examples

### Example 5: In vivo HCT116 xenograft model

Male athymic *nu*/*nu* mice, 6-8 weeks old (about 20 g), were implanted subcutaneously in the right fore flank with about 1×10⁷ cells of the HCT116 (human colon carcinoma) line that had been grown in antibiotic-free medium for at least two passages. About 14-21 days after tumor transplantation, when the tumor weight was about 50-250 mg, the mice were assigned to treatment groups. Test compounds were suspended at 20 mg/mL in 0.55 wt.% aqueous carboxymethylcellulose. Groups of mice were treated with compounds 3, 17, and 46 at 30 mg/Kg intraperitoneally once/day on days 1-5 and 8-12 from the start of treatment, with vehicle control. Tumor growth inhibition was measured 2 days after the last day of treatment. All three compounds tested were active in this assay and showed inhibition of tumor growth compared to vehicle. In addition, compound 46 also showed inhibition of tumor growth when administered orally.

Table 5 listed below shows the biological data as well as mass spectrum analysis for the compounds of Tables 1-4.

**Table 5**

| **Compound #** | **Exact Mass** | **MS(+)** | **VEGFR2 IC50 (nM) (FP)** | **Cytotoxicity HCT116 (µM)** | **Cytotoxicity HL 60 (µM)** |
|---|---|---|---|---|---|
| **1** | 456 | 457 | 1000 | 4 | 0.9 |
| **2** | 474 | * | 2000 | 7 | * |
| **3** | 373 | 374 | 20 | 20 | 3.0 |
| **4** | 388 | 389 | 10 | 6 | 0.2 |
| **5** | 391 | 392 | 30 | 30 | * |
| **6** | 407 | 408 | 20 | >50 | 3.0 |
| **6** | 441 | 442 | 40 | 10 | 0.5 |
| **8** | 407 | 408 | 70 | 20 | 0.3 |
| **9** | 409 | 410 | 300 | 50 | 20.0 |
| **10** | 474 | 475 | 20 | 2 | 0.5 |
| **11** | 436 | 437 | 10 | 3 | * |
| **12** | 406 | 407 | 100 | 40 | 0.1 |
| **13** | 418 | 419 | 200 | >50 | >50 |
| **14** | 418 | 419 | 30 | 20 | 0.3 |
| **15** | 449 | 450 | 400 | 3 | 1.0 |
| **16** | 422 | 423 | 4 | 10 | 1.0 |
| **17** | 402 | 403 | 10 | 1 | 0.3 |
| **18** | 422 | 423 | 30 | 30 | 0.5 |
| **19** | 402 | 403 | 70 | >50 | 0.9 |
| **20** | 456 | 457 | 50 | 50 | 0.7 |
| **21** | 374 | 375 | 200 | >50 | 4.0 |
| **22** | 490 | 491 | 20 | 1 | 1.0 |
| **23** | 374 | 375 | 500 | 10 | * |
| **24** | 374 | 375 | 1000 | 30 | * |
| **25** | 403 | 404 | 300 | 10 | * |
| **26** | 418 | 419 | 40 | 7 | 0.4 |
| **27** | 416 | 417 | 40 | 2 | 4.0 |
| **28** | 416 | 417 | 70 | 8 | 1.0 |
| **29** | 413 | 414 | 60 | >50 | * |
| **30** | 413 | 414 | 400 | >50 | * |
| **31** | 422 | 423 | 60 | 1 | * |
| **32** | 457 | 458 | 30 | 4 | * |
| **33** | 433 | 434 | 50 | >50 | * |
| **34** | 433 | 433 | 200 | | * |
| **35** | 431 | 432 | 100 | >50 | * |
| **36** | 488 | 489 | 300 | 10 | * |
| **37** | 388 | 389 | 200 | 2 | * |
| **38** | 430 | 431 | 200 | >50 | * |
| **39** | 456 | 457 | 20 | 2 | 1.0 |
| **40** | 455 | 456 | 100 | 7 | * |
| **41** | 387 | 388 | 10 | 20 | * |
| **42** | 460 | 461 | 100 | 4 | * |
| **43** | 446 | 447 | 20 | 5 | * |
| **44** | 418 | 419 | 30 | 30 | * |
| **45** | 387 | 388 | 40 | 30 | * |
| **46** | 487 | 488 | 20 | 8 | 0.7 |
| **47** | 470 | 471 | 90 | >50 | * |
| **48** | 373 | 374 | 1000 | * | * |
| **49** | 387 | 388 | 400 | 10 | * |
| **50** | 387 | 388 | 2000 | * | * |
| **51** | 401 | 402 | 900 | 10 | * |
| **52** | 472 | 473 | 500 | 4 | 1.0 |
| **53** | 470 | 471 | 500 | 1 | 0.8 |
| **54** | 432 | 433 | 2000 | * | * |
| **55** | 430 | 431 | 400 | 2 | 0.4 |
| **56** | 446 | 447 | 100 | 3 | 2.0 |
| **57** | 460 | 461 | * | * | * |
| **58** | 402 | 403 | 60 | 4 | 0.4 |
| **59** | 398 | 399 | 300 | 30 | * |
| **60** | 340 | 341 | 80 | >50 | * |
| **61** | 311 | 312 | 300 | 40 | * |
| **62** | 379 | 380 | 20 | >50 | * |
| **63** | 380 | 381 | 70 | 7 | * |
| **64** | 365 | 366 | 300 | >50 | * |
| **65** | 394 | 395 | 40 | 8 | 1.0 |
| **66** | 396 | 397 | 2000 | * | * |
| **67** | 394 | 395 | 300 | 10 | * |
| **68** | 380 | 381 | 200 | 20 | * |
| **69** | 396 | 397 | 400 | 4 | * |
| **70** | 396 | 397 | 2000 | * | * |
| **71** | 467 | 468 | >1000 | * | * |
| **72** | 432 | 433 | 60 | 2 | 0.3 |

| | | | | | |
|---|---|---|---|---|---|
| * - not determined | | | | | |

As to the table above, preferred compounds are those showing one or more of the following: VGRF2 IC₅₀ value of less than 100 nm and/or cytotoxicity in the Cytotoxicity HCT116 assay of 40 µM or less.

### Formulation examples

### Example 6: Formulation for oral administration

A solid formulation for oral administration is prepared by combining the following:
Compound of this invention 25.0% w/w
Magnesium stearate 0.5% w/w
Starch 2.0% w/w
Hydroxypropylmethylcellulose 1.0% w/w
Microcrystalline cellulose 71.5% w/w
and the mixture is compressed to form tablets or filled into hard gelatin capsules containing, for example, 100 mg of the compound of this invention. Tablets may be coated, if desired, by applying a suspension of a film-forming agent (for example, hydroxypropylmethylcellulose), pigment (for example, titanium dioxide), and plasticizer (for example, diethyl phthalate), and drying the film by evaporation of the solvent.

### Example 7. Formulation for IV administration

A formulation for IV administration is prepared by dissolving a compound of this invention, for example as a pharmaceutically acceptable salt, to a concentration of 1% w/v in phosphate-buffered saline; and the solution is sterilized, for example by sterile filtration, and sealed in sterile containers containing, for example, 100 mg of a compound of this invention.

Alternatively, a lyophilized formulation is prepared by dissolving a compound of this invention, again for example as a pharmaceutically acceptable salt, in a suitable buffer, for example the phosphate buffer of the phosphate-buffered saline mentioned above, optionally with the addition of a bulking agent and other pharmaceutically useful excipients, sterilizing the solution and dispensing it into suitable sterile vials, lyophilizing the solution to remove the water, and sealing the vials. The lyophilized formulation is reconstituted by the addition of sterile water, and the reconstituted solution may be further diluted for administration with a solution such as 0.9% sodium chloride intravenous infusion or 5% dextrose intravenous infusion.

While this invention has been described in conjunction with specific embodiments and examples, it will be apparent to a person of ordinary skill in the art, having regard to that skill and this disclosure, that equivalents of the specifically disclosed materials and methods will also be applicable to this invention; and such equivalents are intended to be included within the following claims.

## Claims

1. A compound of formula I: 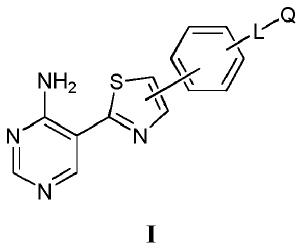 wherein:
L is selected from the group consisting of -NHC(X)-, -C(X)NH-, -NHC(X)NH-, -C(X)-, -C(X)NH-alkylene- and -NHC(X)-alkylene-, where X is oxygen or sulfur; and
Q is selected from the group consisting of alkyl, substituted alkyl, aryl, substituted aryl, heteroaryl, substituted heteroaryl, cycloalkyl, substituted cycloalkyl, heterocyclicand substituted heterocyclic,
or a pharmaceutically acceptable salt thereof.

2. The compound of claim 1, wherein the compound is represented by formula IA:

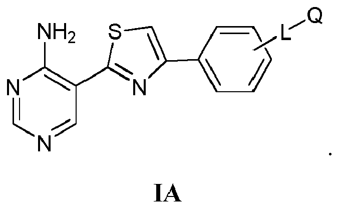

3. The compound of claim 1 or claim 2, wherein Q is selected from the group consisting of aryl, substituted aryl, heteroaryl, and substituted heteroaryl, such as phenyl, substituted phenyl, pyridyl and substituted pyridyl.

4. The compound of any one of claims 1 to 3, wherein the compound is represented by formula IC: 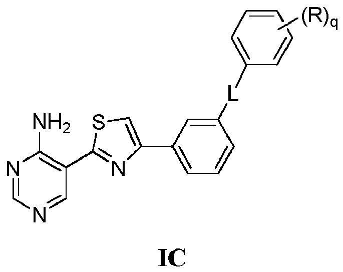 wherein:
q is 0, 1 or 2; and
each R is independently selected from the group consisting of halo, alkyl, substituted alkyl, alkoxy, amino, cyano, nitro, heteroaryl, substituted heteroaryl, carboxy and alkxoxycarbonyl; or
when q is 2, the two R can join to form a ring.

5. The compound of claim 4, wherein L is selected from the group consisting of -NHC(X)-, -C(X)NH-, -NHC(X)NH-, -C(X)NH-alkylene- and -NHC(X)-alkylene-.

6. The compound of claim 4, wherein q is 1 or 2.

7. The compound of any one of claims 1 to 3, wherein the compound is represented by formula ID: 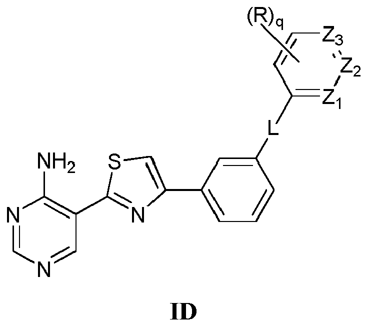 wherein:
q is 0, 1 or 2;
each R is independently selected from the group consisting of halo, alkyl, substituted alkyl, alkoxy, amino, cyano, nitro, heteroaryl, substituted heteroaryl, carboxy and alkxoxycarbonyl; or
when q is 2, the two R can join to form a ring; and
each of Z₁, Z₂, and Z₃ is independently N or CH.

8. The compound of claim 7, wherein L is -NHC(X)-.

9. The compound of claim 7 or claim 8, wherein q is 0 or 1.

10. The compound of any one of claims 7 to 9, wherein R is alkyl.

11. The compound of claim 2, wherein L is selected from the group consisting of NHC(X)-, - NHC(X)NH-, -C(X)- and -NHC(X)-alkylene-, where X is oxygen or sulfur.

12. The compound of claim 5 or claim 11, wherein alkylene is CH₂.

13. A compound selected from the compounds listed in Tables 1-4.

14. A pharmaceutical composition comprising a compound of any one of claims 1-13 and a pharmaceutically acceptable excipient.

15. A compound of any one of claims 1-13 for use as a VEGFR2 kinase inhibitor or as a VEGFR2 kinase inhibitor in the treatment of cancer.

16. The compound for use in the treatment of cancer of claim 15, wherein the compound has cytotoxicity against HCT116 of 40 µM or less.
